(19) **Europäisches Patentamt / European Patent Office / Office européen des brevets**

(11) **EP 4 786 984 A1**

(12) **EUROPEAN PATENT APPLICATION**

(43) Date of publication:
05.08.2026 Bulletin 2026/32

(21) Application number: 25155160.2

(22) Date of filing: 31.01.2025

(51) International Patent Classification (IPC):
$G01N\ 33/579^{(2006.01)}$      $A61M\ 1/00^{(2006.01)}$
$A61P\ 35/00^{(2006.01)}$      $A61P\ 37/00^{(2006.01)}$

(52) Cooperative Patent Classification (CPC):
A61P 35/00; A61M 1/3486; A61M 1/3679;
A61P 37/00

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicants:
• **Bornstein, Stefan**
**01309 Dresden (DE)**
• **Tselmin, Sergey**
**01309 Dresden (DE)**
• **Rodionov, Roman**
**01099 Dresden (DE)**
• **Jarzebska, Natalia**
**01099 Dresden (DE)**
• **King's College London**
**London WC2R 2LS (GB)**

(72) Inventors:
• **Bornstein, Stefan**
**01309 Dresden (DE)**
• **Tselmin, Sergey**
**01309 Dresden (DE)**
• **Rodionov, Roman**
**01099 Dresden (DE)**
• **Jarzebska, Natalia**
**01099 Dresden (DE)**
• **Malik, Afshan**
**London, NW10 5SH (GB)**

(74) Representative: **Hertin und Partner**
**Rechts- und Patentanwälte PartG mbB**
**Kurfürstendamm 63**
**10707 Berlin (DE)**

(54) **MATRIX FOR EXTRACORPOREAL REMOVAL OF MT-DNA FROM BLOOD**

(57)     The invention relates to a matrix configured for use in an extracorporeal blood treatment device, wherein the matrix comprises a solid substrate, to which an agent is immobilized, wherein said agent specifically binds mitochondrial DNA (mtDNA) in the blood or blood plasma of a subject. The invention also relates to methods for preparing the matrix according to the present invention, wherein the oligonucleotide and/or the polypeptide is immobilized on the solid substrate of the matrix by covalent coupling, preferably by crosslinking. The invention further relates to a blood treatment device configured to remove mtDNA from the blood or blood plasma of a person in need thereof in an extracorporeal blood circuit, wherein the device comprises a matrix according to the present invention. The invention further relates to an extracorporeal blood circuit comprising a blood treatment device according to the present invention, wherein the extracorporeal blood circuit comprises means for transporting blood or blood plasma from a patient's vascular system to the blood treatment device at a defined flow rate and means for returning the treated blood or blood plasma back to the patient.

EP 4 786 984 A1

## Description

The invention relates to the fields of molecular biology, medicine, mitochondrial DNA and extracorporeal blood treatment.

The invention relates to a matrix configured for use in an extracorporeal blood treatment device, wherein the matrix comprises a solid substrate, to which an agent is immobilized, wherein said agent specifically binds mitochondrial DNA (mtDNA) in the blood or blood plasma of a subject.

The invention also relates to a method for preparing the matrix according to the present invention, wherein the oligonucleotide and/or the polypeptide is immobilized on the solid substrate of the matrix by covalent coupling, preferably by crosslinking.

The invention further relates to a blood treatment device configured to remove mtDNA from the blood or blood plasma of a person in need thereof in an extracorporeal blood circuit, wherein the device comprises a matrix according to the present invention.

The invention further relates to an extracorporeal blood circuit comprising a blood treatment device according to the present invention, wherein the extracorporeal blood circuit comprises means for transporting blood or blood plasma from a patient's vascular system to the blood treatment device at a defined flow rate and means for returning the treated blood or blood plasma back to the patient.

The invention further relates to an *ex vivo* method for binding mtDNA comprising contacting mtDNA with the matrix according to the present invention.

The invention further relates to an agent that specifically binds mitochondrial DNA (mtDNA) for use in the treatment and/or prevention of a medical condition associated with cell free (cf-) mtDNA, wherein the agent is immobilized to a solid substrate of a matrix, wherein said matrix is configured for specifically binding and removing cf-mtDNA from the blood or blood plasma of a subject, wherein said matrix is present in a blood treatment device and wherein said device is present in an extracorporeal blood circuit transporting blood or blood plasma from a patient's vascular system to the blood treatment device and returning the treated blood or blood plasma back to the patient.

## BACKGROUND OF THE INVENTION

Mitochondria are cellular organelles containing an extranuclear circular genome known as mitochondrial DNA (mtDNA), which shares structural similarities with bacterial DNA, particularly in its circular form and methylation status. Within cells, mtDNA is typically organized as nucleoids by binding to mitochondrial transcription factor A (TFAM) and is located in the inner mitochondrial membrane. mtDNA encodes essential subunits of the electron transport chain, critical for oxidative phosphorylation, the process through which cells generate most of their ATP energy. The number of mtDNA copies per cell varies depending on cell type, reflecting cell-specific energy demands. For example, mature oocytes contain approximately 100,000 copies, skeletal muscle fibers contain thousands, and whole blood contains 10-100 copies. mtDNA copy number can also vary in response to physiological signals or disease conditions, with the highest concentrations observed in energy-intensive organs such as the brain, heart, and kidneys.

Under normal physiological conditions, mtDNA is continuously replicated, and damaged mtDNA is removed through targeted degradation as part of the mitochondrial life cycle. However, disrupting this process can impair the degradation of damaged mtDNA, leading to its release into the cytosol or peripheral circulation. Once released, cell-free (cf) mtDNA, due to its bacterial-like structure, can activate innate immune pathways, such as the Toll-like receptor 9 (TLR-9) pathway, triggering TNF-alpha activation and downstream inflammatory responses.

The presence of cf-mtDNA in the bloodstream presents a significant challenge due to its ability to mimic bacterial DNA and trigger the body's innate immune responses. This mimicry is primarily due to its unmethylated CpG motifs, which are recognized by the immune system's pattern recognition receptors, which activate inflammatory pathways. The resultant inflammation can exacerbate existing conditions and contribute to disease progression, making managing cf-mtDNA levels a crucial aspect of mitigating its pathological effects. Consequently, a targeted approach to remove cf-mtDNA could potentially alleviate these inflammatory responses, offering a new avenue for the treatment of various diseases associated with mitochondrial dysfunction.

Increasing evidence suggests that alterations in mtDNA levels, particularly circulating cf-mtDNA, can serve as biomarkers of mitochondrial dysfunction. Changes in cf-mtDNA levels have been observed in a wide range of diseases, including cancer, HIV-associated complications, and metabolic disorders. Elevated cf-mtDNA levels have also been associated with an increased risk of diabetes and its complications, cardiovascular disease, neurodegenerative diseases, COVID-19 pathogenesis, and muscle-wasting disorders. Furthermore, cf-mtDNA has been implicated as a risk marker in conditions such as HIV infection and inflammation, pregnancy, late-life depression, brain diseases, and mortality in critical care patients.

Furthermore, it has been shown that cf-mtDNA functions is a pathogenic molecule capable of inducing inflammation. In chronic conditions, cf-mtDNA can drive low-grade inflammation, while in acute settings, it can cause severe inflammatory responses leading to heart failure or multi-organ failure. Despite its well-established role in disease pathology, no published or known method currently exists for the targeted removal of circulating cf-mtDNA.

Current approaches to managing diseases linked with cf-mtDNA focus on addressing symptoms rather than the underlying cause. For instance, treatments for inflammation-related conditions often involve anti-inflammatory drugs or immunosuppressants, which can have broad effects and potential side effects due to their lack of specificity.

WO2021/108637A1 discloses compositions and methods for treating diseases and conditions by depleting mitochondrial or genomic DNA from circulation. It involves proteins that bind to mtDNA and/or gDNA and employs an Fc fragment from IgG receptor gamma (FcgRIIb) for therapeutic purposes. The removal of mtDNA is based on the systemic administration of the proteins,

The systemic administration of therapeutic proteins for mtDNA targeting, as described in the prior art, can have significant drawbacks. For example, non-specific targeting can lead to unintended interactions with other biomolecules, reducing efficacy and increasing side effects. Additionally, limited control over distribution of the active agent may result in uncontrolled protein delivery, making it challenging to achieve therapeutic concentrations at the target site and minimize off-target exposure.

Proteins also might face a short circulatory half-life, requiring frequent dosing, which increases the treatment burden and reduces compliance. Metabolic degradation can further reduce their effectiveness, necessitating higher doses. Moreover, systemic therapies are hindered by the complexity and cost of protein production and purification, limiting accessibility.

Thus, despite advances in the field, there is an unmet need for the development of a therapeutic strategy or device capable of specifically capturing and removing cf-mtDNA from human plasma, ensuring precise delivery and action at the intended site while minimizing interactions with non-target biomolecules.

## SUMMARY OF THE INVENTION

In light of the prior art, the technical problem underlying the invention was providing alternative or improved means for a matrix configured for use in the extracorporeal blood treatment. The present invention seeks to provide such means while avoiding the disadvantages known in the prior art.

One objective of the present invention was to develop a therapeutic strategy or device capable of specifically capturing and removing circulating cell-free mitochondrial DNA (cf-mtDNA) from human plasma.

A further objective of the invention was to provide alternative or improved means for reducing the levels of cf-mtDNA in the bloodstream, addressing the root cause of inflammation rather than just alleviating symptoms.

A further objective of the invention was to provide alternative or improved means for reducing the levels of cf-mtDNA in the bloodstream, while reducing non-specific and off-target effects, and ensuring precise delivery and action at the intended site, minimizing interactions with non-target biomolecules.

A further objective of the invention was to provide alternative or improved means for reducing the levels of cf-mtDNA in the bloodstream, mitigating potential unwanted immune responses and metabolic degradation, thereby enhancing the durability and efficacy of the treatment.

A further objective of the invention was to provide an improved method for assessing cf-mtDNA levels in plasma as a diagnostic tool.

A further objective of the invention was to provide a preventive approach for managing diseases associated with cf-mtDNA by maintaining its levels within a physiological range, potentially reducing the incidence and/or severity of inflammatory and cf-mtDNA-related disorders.

These problems are solved by the features of the independent claims. Preferred embodiments of the present invention are provided by the dependent claims.

The present invention, in one aspect, relates to a matrix configured for use in an extracorporeal blood treatment device, wherein the matrix comprises a solid substrate, to which an agent is immobilized, wherein said agent specifically binds mitochondrial DNA (mtDNA) in the blood or blood plasma of a subject.

To the best of the inventors' knowledge, the matrix disclosed herein represents the first solid substrate matrix incorporating an immobilized agent capable of specifically binding to mitochondrial DNA. The inventive aspect of this disclosure lies in the novel approach of employing the matrix to selectively target and remove mtDNA from blood or plasma.

It was particularly surprising that the immobilized agent within the matrix exhibits a specific binding affinity for mtDNA in blood, enabling its effective removal. This targeted binding and elimination of mtDNA minimizes off-target interactions. Furthermore, the matrix can advantageously be customized by incorporating various immobilized agents, thereby allowing the selective binding of different forms of mtDNA. This adaptability confers potential applicability across diverse patient populations, enhancing the invention's clinical utility and efficacy.

In embodiments, the matrix is configured to bind and remove cell-free mitochondrial DNA (cf-mtDNA) from human blood or blood plasma by extracorporeal treatment.

In embodiments, the matrix binds and removes cf-mtDNA from human blood or blood plasma by extracorporeal treatment. In embodiments, the solid substrate binds and removes cf-mtDNA from human blood or blood plasma by extracorporeal treatment. In embodiments, the agent binds and removes cf-mtDNA from human blood or blood plasma by extracorporeal treatment.

Proposed methods for removing cell-free mitochondrial DNA from blood or plasma exhibit several significant drawbacks, as known in the art. A primary limitation is non-specific binding, wherein adsorbents used to capture cf-mtDNA may lack specificity, resulting in reduced efficacy. Furthermore, the presence of other biomolecules in the blood, such as proteins, lipids, and cell-free nuclear DNA, can compete with mtDNA for binding sites, further impairing the specificity and efficiency of the adsorption process. Another critical limitation is the potential for DNA damage inherent in some methodologies. For instance, adsorption techniques often require harsh conditions, such as elevated temperatures, extreme pH levels, or strong chemical agents, which can degrade or denature the DNA. These challenges underscore the need for improved technologies capable of specifically and efficiently removing cf-mtDNA under biologically compatible conditions while avoiding off-target effects.

The matrix-based or column-based approach described in the present application represents a significant and innovative advancement over the prior art (such as WO2021/108637A1) by employing extracorporeal removal of mtDNA during blood treatment. By confining the treatment to an extracorporeal device, such as a column, systemic exposure to mtDNA-binding agents is minimized, thereby reducing potential side effects associated with their systemic administration. The immobilization of oligonucleotides or polypeptides within the column ensures high specificity and efficiency in targeting mtDNA, as these agents remain fixed and can repeatedly capture mtDNA during blood flow. Structural stability and durability are achieved through covalent coupling techniques, enabling the column to withstand multiple treatment cycles without losing efficacy. Moreover, the column's design allows customization with different binding agents to target specific mtDNA forms, offering a tailored and adaptive treatment approach. The system also provides a controlled environment for mtDNA removal, precisely regulating critical parameters such as flow rate, temperature, and binding agent concentration. These conditions are inherently challenging to control in systemic treatments. Furthermore, the matrix-based approach supports scalability and high-throughput applications, accommodating various blood volumes and flow rates, making it particularly advantageous for clinical settings.

One main advantage of the present invention lies in its ability to facilitate the binding and removal of cf-mtDNA from human blood or blood plasma through extracorporeal treatments. This approach enables the selective targeting and extraction of cf-mtDNA while maintaining compatibility with physiological conditions, thereby minimizing potential damage to other biomolecules or cells. The extracorporeal application further allows for treating blood or plasma outside the body, reducing systemic exposure to potentially harmful byproducts and enhancing the precision of cf-mtDNA removal. Hence, the present invention offers a significant improvement over proposed methods, addressing key challenges such as non-specific binding and biomolecule competition while ensuring efficient and safe cf-mtDNA clearance.

In embodiments, an oligonucleotide and/or a polypeptide is/are immobilized on a solid substrate of the matrix according to the present invention. In embodiments, an oligonucleotide is immobilized on a solid substrate of the matrix according to the present invention. In embodiments, a polypeptide is immobilized on a solid substrate of the matrix according to the present invention.

In embodiments, the agent comprises an oligonucleotide and/or a polypeptide that specifically binds mtDNA.

In embodiments, the agent comprises an oligonucleotide that specifically binds (or hybridizes to) mtDNA. In embodiments, the agent comprises a polypeptide that specifically binds mtDNA. In embodiments, the agent comprises an oligonucleotide and a polypeptide that specifically binds mtDNA.

In embodiments, the oligonucleotide binds and depletes circulating mtDNA from circulation. In embodiments, the polypeptide binds and depletes circulating mtDNA from circulation.

In embodiments, the present invention provides for a nucleic acid encoding the polypeptide of the present invention as described herein. In embodiments, the present invention provides for a nucleic acid encoding the oligonucleotide of the present invention as described herein.

In embodiments, the present invention provides for a cell for producing the polypeptide of the present invention as described herein. In embodiments, the present invention provides for a cell for producing the oligonucleotide of the present invention as described herein.

In embodiments, the agent comprises a combination of an oligonucleotide and a polypeptide.

In embodiments, the agent comprises multiple oligonucleotides of different nucleic acid sequences, each hybridizing to an mtDNA sequence.

Advantageously, the use of multiple oligonucleotides and/or polypeptides with distinct nucleic acid sequences facilitates binding to diverse mtDNA sequences, thereby enhancing the likelihood of capturing all relevant variants.

In embodiments, the oligonucleotide is a single-stranded DNA (ssDNA) oligonucleotide.

In embodiments, the ssDNA oligonucleotide hybridizes to a complementary sequence on the mtDNA. In embodiments, the ssDNA oligonucleotides hybridizes to a complementary sequence on the mtDNA, thereby depleting said mtDNA.

ssDNA oligonucleotides offer significant advantages in binding to target sequences, particularly mtDNA. ssDNA oligonucleotides can hybridize with high specificity to complementary sequences on mtDNA, ensuring selective binding while minimizing off-target effects. Their inherent flexibility allows for efficient access and binding to complementary sequences, even within complex or structured regions of the target DNA. Additionally, ssDNA exhibits faster hybridization kinetics than double-stranded DNA, which is particularly beneficial in applications requiring rapid binding, such as extracorporeal blood treatment devices with limited contact time. ssDNA oligonucleotides are also relatively simple to synthesize and can be chemically modified to enhance stability, improve binding affinity, or enable attachment to solid supports in a matrix, allowing for application-specific customization. Furthermore, ssDNA reduces design complexity by eliminating the need for denaturation and streamlining workflows in processes such as mtDNA removal. Collectively, these attributes make ssDNA oligonucleotides a versatile and valuable tool for precise and efficient binding to specific DNA sequences, particularly in therapeutic and diagnostic applications targeting mtDNA.

In embodiments, the oligonucleotide has a length of 10 to 50 nt, preferably 12 to 30 nt.

In embodiments, the oligonucleotide has a length of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nt, preferably 12, 13, 14, 15, 16, 17, 18, 19, 20, 21, 22, 23, 24, 25, 26, 27, 28, 29, 30 nt.

It has been demonstrated that this range of oligonucleotide lengths ensures effective hybridization and binding specificity to the target sequence under physiological conditions.

In embodiments, the oligonucleotide is obtained by synthesizing multiple mtDNA sequences of a human mtDNA genome, wherein the mtDNA sequences are synthesized as overlapping amplicons from the sense and antisense strand of said human mtDNA genome.

In embodiments, the polypeptide is obtained by synthesizing multiple mtDNA sequences of a human mtDNA genome, wherein the mtDNA sequences are synthesized as overlapping amplicons from the sense and antisense strand of said human mtDNA genome.

In embodiments, the mtDNA sequence is synthesized as an overlapping amplicon from the sense and antisense strand of a human mtDNA genome. In embodiments, the amplicon provides coverage of the entire mitochondrial genome.

In one embodiment, the oligonucleotide captures mtDNA fragments regardless of their specific sequence and/or orientation. In one embodiment, the polypeptide captures mtDNA fragments regardless of their specific sequence and/or orientation.

In one embodiment, mtDNA sequences spanning the 16.5 kb of the human mtDNA genome are synthesized as 15, 16, 17, 18, 19, 20 bp overlapping amplicons from a sense and an antisense strand.

In embodiments, the oligonucleotide is immobilized on Sepharose beads by crosslinking.

The use of overlapping amplicons synthesized from both strands of the mtDNA beneficially provides comprehensive coverage of the entire mitochondrial genome, ensuring the capture of all relevant mtDNA fragments regardless of their specific sequence or orientation. This dual-strand approach increases binding efficiency by offering multiple hybridization opportunities for any region, thereby enhancing the probability of successful mtDNA capture. Additionally, targeting both sense and antisense strands improves specificity and sensitivity, enabling precise discrimination between mtDNA and non-target nucleic acids while minimizing off-target effects. The redundancy inherent in overlapping sequences further enhances the system's robustness against mutations or sequence variations within mtDNA, ensuring consistent performance even in the presence of genetic variability.

In embodiments, the polypeptide and/or oligonucleotide specifically binds to human mtDNA.

In embodiments, the polypeptide specifically binds to human mtDNA, and is a protein selected from the group consisting of TFAM, POLG, mtSSB (mitochondrial single stranded DNA binding protein), Twinkle (mtDNA helicase), C17orf80, SSBP1, POLRMT, DHX30, DDX28,HADHA, SHMT2, POLG, POLG2, TFB2M, TFB1M, MTERF, PE01, SUPV3L1, HSPA1, HSPD1, PHB2, ATAD3, ALC25A5, CPT1A, DNAJ3, NDUFS1, LRPPRC, ATB5B, SLC25A5, VDAC1, VDAC2, NDUFA9, CLPX, HADHB, TOP1MT, TUFM, NDUFS3, CPS1, PDIP38, DBT, LRRC59, MGC5352, IMMT, KTN1, RPN1, C17orf80, Mgm101p, MTERF1, LONP1 and MTERF2, preferably TFAM, POLG, mtSSB (mitochondrial single stranded DNA binding protein), Twinkle (mtDNA helicase), C17orf80, and/or mtDNA-binding fragments thereof.

In embodiments, the matrix of the present invention incorporates TFAM, POLG, mtSSB (mitochondrial single stranded DNA binding protein), Twinkle (mtDNA helicase), C17orf80, SSBP1, POLRMT, DHX30, DDX28,HADHA, SHMT2, POLG, POLG2, TFB2M, TFB1M, MTERF, PE01, SUPV3L1, HSPA1, HSPD1, PHB2, ATAD3, ALC25A5, CPT1A, DNAJ3, NDUFS1, LRPPRC, ATB5B, SLC25A5, VDAC1, VDAC2, NDUFA9, CLPX, HADHB, TOP1MT, TUFM, NDUFS3, CPS1, PDIP38, DBT, LRRC59, MGC5352, IMMT, KTN1, RPN1, C17orf80, Mgm101p, MTERF1, LONP1 and/or MTERF2 and/or derivatives and/or fragments thereof as binding agents to capture and remove mtDNA.

In embodiments, TFAM, POLG, mtSSB (mitochondrial single stranded DNA binding protein), Twinkle (mtDNA helicase), C17orf80, SSBP1, POLRMT, DHX30, DDX28,HADHA, SHMT2, POLG, POLG2, TFB2M, TFB1M, MTERF, PE01, SUPV3L1, HSPA1, HSPD1, PHB2, ATAD3, ALC25A5, CPT1A, DNAJ3, NDUFS1, LRPPRC, ATB5B, SLC25A5, VDAC1, VDAC2, NDUFA9, CLPX, HADHB, TOP1MT, TUFM, NDUFS3, CPS1, PDIP38, DBT, LRRC59, MGC5352, IMMT, KTN1, RPN1, C17orf80, Mgm101p, MTERF1, LONP1 and/or MTERF2 and/or derivatives and/or fragments thereof are immobilized on the matrix, preferably to serve as an active agent that specifically binds to mtDNA.

In embodiments, the polypeptide is produced in a bacterial system using a vector suitable for overexpression.

Hence, by leveraging the intrinsic DNA-binding properties of the aforementioned proteins, the matrix can effectively capture cf-mtDNA as the blood or plasma flows through the extracorporeal device. In the context of the present invention, the polypeptides derived from these proteins are used for their DNA-binding properties. When integrated into the matrix, these polypeptides engage in direct molecular interactions with cf-mtDNA present in the blood or plasma, resulting in the sequestration of cf-mtDNA. This mechanism provides a therapeutic advantage by effectively reducing cf-mtDNA levels, which, in turn, may attenuate inflammatory responses and mitigate the risk of diseases associated with elevated cf-mtDNA concentrations.

In one aspect, the invention relates to a method for preparing the matrix according to any one of the preceding claims, wherein the oligonucleotide and/or the polypeptide according to the present invention is immobilized on the solid substrate of the matrix by covalent coupling, preferably by crosslinking, for example by EDC/NHS (1-ethyl-3-(3-dimethylamino) propyl carbodiimide/N-hydroxysulfosuccinimide) treatment.

In embodiments, the agent comprising an oligonucleotide and/or a polypeptide is immobilized on the solid substrate of the matrix by covalent coupling. In one embodiment, the agent comprising an oligonucleotide and/or a polypeptide is immobilized on the solid substrate of the matrix by EDC/NHS, carbodiimide coupling, succinimidyl ester coupling, sulfhydryl coupling resin, amino coupling resin, NHS-activated resin, silane coupling, thiol-maleimide coupling, photolithography, biotin-streptavidin interaction, and/or click chemistry.

In one embodiment, the agent comprising an oligonucleotide and/or a polypeptide is immobilized on the solid substrate of the matrix by crosslinking. In one embodiment, the agent comprising an oligonucleotide and/or a polypeptide is immobilized on the solid substrate of the matrix by glutaraldehyde, Bis(sulfosuccinimidyl) suberate (BS3), and/or UVA-irradiation.

In a preferred embodiment, the agent comprising an oligonucleotide and/or a polypeptide is immobilized on the solid substrate of the matrix by EDC/NHS.

Advantageously, using covalent coupling and crosslinking techniques in constructing the matrix provides a robust and durable platform that preserves its structural integrity and functional performance over extended periods of use. These chemical bonding methods ensure that the immobilized agents remain securely attached to the substrate, preventing leaching or degradation under operational conditions. This durability is particularly advantageous for extracorporeal treatments, where consistent performance and reliability are critical. By maintaining the matrix's binding capacity and specificity, covalent coupling and crosslinking contribute to the long-term efficacy and stability of the invention, making it suitable for repeated or sustained applications.

In one aspect, the invention relates to a blood treatment device configured to remove mtDNA from the blood or blood plasma of a person in need thereof in an extracorporeal blood circuit, wherein the device comprises a matrix according to the present invention.

In one aspect, the invention relates to an extracorporeal blood circuit, comprising a blood treatment device according to the present invention, wherein the extracorporeal blood circuit comprises means for transporting blood or blood plasma from a patient's vascular system to the blood treatment device at a defined flow rate and means for returning the treated blood or blood plasma back to the patient.

In embodiments, the extracorporeal blood circuit in which the blood treatment device is located further comprises a plasma separator, such as a plasma filter or centrifuge-based plasma separation system or combination thereof, which allows for the separation of plasma from the blood, and wherein the blood treatment device is located downstream of a plasma outlet of the plasma separator.

In one aspect, the invention relates to an *ex vivo* method for binding mtDNA comprising contacting mtDNA with the matrix according to the present invention.

The *ex vivo* method for binding mtDNA presents several distinct advantages. First, it operates in a controlled environment, enabling precise regulation of binding conditions, such as pH, temperature, and ionic strength. This controlled setting enhances the efficiency of mtDNA capture and removal, reducing variability and off-target interactions. Second, the *ex vivo* approach is a robust platform for testing and optimization, allowing for the evaluation of various binding agents, matrix configurations, or operational conditions before clinical application. This capability supports the customization of treatments, facilitating adaptation to the specific needs of individual patients or the demands of particular clinical strategies.

In one aspect, the invention relates to an agent that specifically binds mitochondrial DNA (mtDNA), as described in the present invention, for use in the treatment and/or prevention of a medical condition associated with cf-mtDNA, wherein the agent is immobilized to a solid substrate of a matrix, wherein said matrix is configured for specifically binding and removing cf-mtDNA from the blood or blood plasma of a subject, wherein said matrix is present in a blood treatment device according to the present invention, and wherein said device is present in an extracorporeal blood circuit transporting blood or blood plasma from a patient's vascular system to the blood treatment device and returning the treated blood or blood plasma back to the patient.

In one aspect, the invention relates to an agent for use according to the present invention, wherein the medical condition is selected from the group consisting of an inflammatory disease, a metabolic disorder, a cardiovascular disease, an autoimmune disease, neurodegeneration, an infection, cancer and a psychological disorder.

The various aspects of the invention are unified by, benefit from, are based on and/or are linked by the structural and/or functional features, including functional properties and beneficial technical effects of the matrix configured for use in an extracorporeal blood treatment device described herein. The features disclosed in the context of the matrix also apply to and are considered disclosed in the context of the blood treatment device and vice versa. Any features disclosed in any further aspects of the invention, such as the method for preparing the matrix, the blood treatment device, the extracorporeal blood circuit, the ex vivo method, and the agent are also considered disclosed in the context of the inventive matrix and vice versa.

## DETAILED DESCRIPTION OF THE INVENTION

The present invention relates to a matrix configured for use in an extracorporeal blood treatment device, wherein the matrix comprises a solid substrate, to which an agent is immobilized, wherein said agent specifically binds mitochondrial DNA (mtDNA) in the blood or blood plasma of a subject.

The "matrix" as used herein refers to a structured system or material designed for use in an extracorporeal blood treatment device, where it serves as a platform for immobilizing agents that specifically bind to mtDNA. This matrix comprises a solid substrate onto which oligonucleotides or polypeptides are covalently attached. These binding agents are configured to capture and remove cell-free mtDNA from blood or blood plasma as it passes through the extracorporeal circuit. The matrix's design ensures effective hybridization and capture of mtDNA, thereby facilitating its removal from circulation to reduce inflammation and mitigate associated disease risks. The matrix is integral to the device's function, enabling targeted therapeutic intervention for conditions linked to elevated levels of cf-mtDNA.

In one embodiment, the matrix comprises a solid substrate to which an agent is immobilized and wherein said agent specifically binds mtDNA.

Herein, an "agent" refers to the active component within a matrix used in an extracorporeal blood treatment device, specifically designed to bind and remove mtDNA from the blood or plasma. In the present invention, agents include oligonucleotides and/or polypeptides that are immobilized on a solid substrate. These agents are selected for their specific affinity to mtDNA, enabling them to hybridize with or bind to mtDNA through complementary base pairing or protein-DNA interactions.

As used herein, a "solid substrate" refers to the physical support or material within the matrix of an extracorporeal blood treatment device that provides a stable and durable platform for immobilizing binding agents such as oligonucleotides or polypeptides. This substrate is typically composed of a biocompatible material that can withstand the conditions of blood flow and interaction without degrading or negatively affecting the blood components. The solid substrate facilitates the covalent attachment of these agents, ensuring that they remain fixed in place while allowing for effective hybridization with and capture of mtDNA from the blood or plasma. Agent

"Mitochondrial DNA (mtDNA)" is a small, circular, or linear genetic molecule located within the mitochondria, distinct from the nuclear genome, that encodes essential components for oxidative phosphorylation (OXPHOS). In most eukaryotes, mtDNA comprises 37 genes, including 13 proteins, 22 transfer RNAs (tRNAs), and two ribosomal RNAs (rRNAs), necessary for ATP production. Unlike nuclear DNA, mtDNA is maternally inherited and lacks introns, with its transcription and replication processes regulated by specific promoters in the displacement loop (D-loop). Variations in mtDNA structure exist across species, ranging from linear forms in unicellular organisms to complex configurations with introns or plasmid-like structures. mtDNA mutations contribute to inherited disorders, age-related pathologies, and degenerative diseases, and their heteroplasmic distribution influences disease severity. mtDNA's high mutation rate and maternal inheritance have applications in evolutionary studies and genealogy. Additionally, its functional interplay with nuclear DNA is vital for mitochondrial and cellular homeostasis, underscoring its dual dependency and evolutionary retention of key genes.

"Cell-free mitochondrial DNA (cf-mtDNA)," also known as circulating mitochondrial DNA (ccf-mtDNA), consists of mitochondrial DNA fragments released into biofluids such as plasma or serum during cellular stress, damage, or physiological processes. Unlike mtDNA confined within organelles, cf-mtDNA exists in various encapsulated forms, including extracellular vesicles, platelets, or whole mitochondria, influencing its accessibility to immune receptors. It is dynamically regulated, exhibiting rapid inducibility and substantial intra-individual variability, and its levels are associated with stress responses, psychopathology, and conditions like trauma, inflammation, cancer, myocardial infarction, and sepsis. Elevated cf-mtDNA levels are predictive of mortality in critically ill patients, underscoring its role as a biomarker of physiological stress. Cf-mtDNA, traditionally considered pro-inflammatory due to its bacterial origin, triggers immune responses via receptor-mediated pathways; however, emerging evidence challenges this view, suggesting potential roles in cell-to-cell mitochondrial transfer and other non-inflammatory functions.

In embodiments, mtDNA, preferably cf-mtDNA, is removed from human blood or plasma by the matrix of the present invention. Several methods are known in the art to measure mtDNA in blood or plasma. Non-limiting methods include qPCR, dPCR, NGS, Southern Blot, ELISA, FISH. The methods vary in their application, sensitivity and specificity. The choice of the methods depends on the specific research or diagnostic requirements, and can be determined by a skilled person in the art.

The terms "mitochondrial DNA (mtDNA)" and "cell-free mitochondrial DNA (cf-mtDNA)" may be used interchangeably within the context of the present disclosure. Accordingly, any embodiments or descriptions pertaining to mtDNA are intended to encompass cf-mtDNA as well, unless explicitly stated otherwise.

"Oligonucleotides" are short, single- or double-stranded DNA or RNA molecules composed of nucleotide residues, often designed and synthesized for specific sequences to support various applications, e.g., in molecular biology, medicine, and diagnostics. They are manufactured, e.g., through solid-phase chemical synthesis, employing phosphoramidite chemistry to add nucleotides in a 3' to 5' direction via iterative synthetic cycles, with purification methods such as HPLC used to isolate products of desired sequences. In their natural form, oligonucleotides function in gene expression regulation, as seen in microRNAs, or as intermediates in nucleic acid degradation. Their ability to hybridize sequence-specifically underpins their utility as probes in techniques such as PCR, DNA microarrays, and fluorescent in situ hybridization. Chemical modifications of oligonucleotides enhance their stability, specificity, and pharmacological properties. Backbone alterations, like phosphorothioate linkages, provide nuclease resistance, while 2'-sugar modifications such as 2'-O-methyl and 2'-O-methoxyethyl groups increase target-binding affinity and reduce off-target interactions. Fluorescent and other functional modifications facilitate structural and interaction studies. As used herein, an oligonucleotide is a short strand of nucleic acids, typically composed of a sequence of 10 to 50 nucleotides, designed to be complementary to specific regions of mtDNA. In the present invention, oligonucleotides are synthesized to bind to target sequences of cf-mtDNA present in the blood or plasma. These oligonucleotides are immobilized on a solid substrate within a matrix used in an extracorporeal blood treatment device. Thus, their primary function is to hybridize with mtDNA through complementary base pairing, facilitating its capture and subsequent removal from the circulation.

"Polypeptides" are long, continuous, unbranched chains of amino acids linked by peptide bonds, forming the structural basis for proteins when their molecular mass exceeds 10,000 Da. Polypeptides are synthesized through ribosomal translation or enzymatic processes and consist of amino acid residues connected via amide bonds, with an N-terminal (amine group) and a C-terminal (carboxyl group). These molecules may undergo post-translational modifications such as phosphorylation, glycosylation, or disulfide bond formation, enhancing their functional diversity and stability. Peptides and polypeptides are classified by their biological sources and functions, encompassing signaling peptides, antimicrobial peptides, and neurotrophic peptides. Ribosomal peptides function as hormones, signaling molecules, or antibiotics (e.g., microcins and bacteriocins), while modular enzyme complexes synthesize nonribosomal peptides and often display cyclic or hybrid structures. Polypeptides engage in numerous biochemical interactions, mediating processes like cell signaling and immune modulation. As used herein, a polypeptide is a linear chain of amino acids linked by peptide bonds, typically comprising a segment or fragment of a protein known for its ability to bind mtDNA. Within the present invention, polypeptides are derived from proteins with a natural affinity for mtDNA, such as TFAM or other mitochondrial-associated proteins. These polypeptides are immobilized on a solid substrate within the matrix of an extracorporeal blood treatment device. Their primary function is to specifically interact with and capture cf-mtDNA from the blood or plasma, thereby facilitating its removal.

Herein, "nucleic acid" may preferably refer to DNA (deoxyribonucleic acid), gDNA (genomic deoxyribonucleic acid), RNA (ribonucleic acid), gRNA (genomic ribonucleic acid), mRNA (messenger ribonucleic acid) and cDNA (complementary deoxyribonucleic acid synthesized from RNA template), or any combination thereof, further also encompassing L-DNA and L-RNA forming so-called mirror-images of the naturally occurring conformation of DNA. As used herein, "nucleic acid" shall mean any nucleic acid molecule, including, without limitation, DNA, RNA, and hybrids or modified variants thereof, including L-DNA and L-RNA. As used herein, a nucleic acid sequence refers to the precise order of nucleotides within a segment of DNA or RNA utilized to bind mtDNA. Furthermore, nucleic acid sequences are specifically designed and synthesized as oligonucleotides to be complementary to target regions of cf-mtDNA. These sequences are immobilized on a solid substrate within a matrix used in an extracorporeal blood treatment device. These nucleic acid sequences hybridize with mtDNA through complementary base pairing, effectively capturing and removing cf-mtDNA from the blood or plasma.

"Hybridization" is the process by which two complementary strands of nucleic acids, such as DNA or RNA, pair with each other to form a double-stranded molecule. This process occurs through the formation of hydrogen bonds between complementary nucleotide bases: adenine (A) pairs with thymine (T) in DNA or with uracil (U) in RNA, and cytosine (C) pairs with guanine (G). Hybridization is a fundamental principle in molecular biology techniques, as it allows for the specific binding of nucleic acid sequences, enabling applications such as DNA sequencing, PCR amplification, and molecular diagnostics, where precise and selective interactions between nucleic acid strands are required. In the context of the invention, hybridization is utilized to enable the binding of synthesized oligonucleotides or amplicons to target mtDNA sequences, facilitating their capture and removal during therapeutic treatments. As used herein, "hybridization" refers to the specific process by which synthesized oligonucleotides or overlapping amplicons bind to complementary sequences of mtDNA present in a sample. This process involves pairing complementary nucleotide bases between the ssDNA oligonucleotides or amplicons and the target mtDNA strands. The formation of stable hydrogen bonds between these complementary bases allows for the precise and selective capture of mtDNA by the matrix. This targeted hybridization is crucial for effectively removing cell-free mtDNA during extracorporeal blood treatment, enhancing the specificity and efficiency of the therapeutic intervention aimed at reducing inflammation and associated disease risks linked to elevated levels of mtDNA in the bloodstream.

In embodiments, multiple oligonucleotides hybridize to an mtDNA sequence. In embodiments, multiple polypeptides hybridize to an mtDNA sequence.

"ssDNA" or single-stranded DNA refers to a DNA molecule that consists of a single strand of nucleotides, unlike the typical double-stranded DNA structure found in cellular organisms. Each nucleotide in ssDNA is composed of a sugar, phosphate group, and one of four nitrogenous bases: adenine (A), cytosine (C), guanine (G), or thymine (T). In the context of the present invention, ssDNA is used for precise binding or hybridization to a complementary DNA sequence. In the invention, ssDNA oligonucleotides are utilized to bind and remove mtDNA from the bloodstream.

In embodiments, an ssDNA oligonucleotide binds and removes an mtDNA from the bloodstream.

As used herein, "synthesizing multiple mtDNA sequences" refers to generating a series of DNA fragments corresponding to various regions of the mtDNA genome. This involves creating precise and specific sequences that collectively cover the entire mtDNA, including both the sense and antisense strands. The synthesis process ensures that these sequences are designed to overlap, forming overlapping amplicons to achieve comprehensive coverage. This comprehensive synthesis is crucial for effectively binding and removing mtDNA in therapeutic applications, such as in the matrix used for extracorporeal blood treatment.

As used herein, an "overlapping amplicon" refers to a segment of DNA that is synthesized to overlap with adjacent segments, covering a specific region of the mtDNA genome. These amplicons are designed to be partially redundant, with each amplicon sharing a portion of its sequence with its neighboring amplicons. This overlap ensures comprehensive coverage and binding potential across the target mtDNA sequence, allowing for effective capture and hybridization in various binding and therapeutic applications. In this invention, overlapping amplicons are used to ensure that both the sense and antisense strands of the mtDNA are effectively targeted, enhancing the specificity and efficiency of mtDNA removal in an extracorporeal treatment setting.

According to the invention, the "immobilization" of a protein to a support providing a matrix that can be used in a device refers to a non-covalent or covalent interaction that holds two molecules together. According to one embodiment of the invention, the expression refers to a covalent interaction, i.e., covalently bound enzymes. Non-covalent interactions include but are not limited to, hydrogen bonding, ionic interactions among charged groups, van der Waals interactions, and hydrophobic interactions among non-polar groups. One or more of these interactions can mediate the binding of two molecules to each other. Binding may otherwise be specific, selective, or unspecific.

Various known methods can be used to immobilize the enzyme to the support and/or matrix, according to the invention. Such immobilization is preferably specific or selective because it immobilizes the enzyme, whereas other proteins and components in blood, blood plasma, or a sample thereof (in vitro) are not immobilized significantly.

A "covalent bond" is a chemical bond in which electrons are exchanged to form electron pairs between atoms; in chemistry, it refers to the interatomic bond formed by sharing electron pairs between two atoms. In the context of the present invention, "covalent coupling" refers to the chemical process by which binding agents, such as oligonucleotides or polypeptides, are permanently attached to a solid substrate within the matrix of the present invention. Non-limiting examples of covalent coupling methods are by EDC/NHS, carbodiimide coupling, succinimidyl ester coupling, silane coupling, thiol-maleimide coupling, photolithography, biotin-streptavidin interaction, or click chemistry. This process involves forming stable covalent bonds between the functional groups of the binding agents and the substrate, ensuring that these agents remain securely immobilized during treatment. Covalent coupling is essential for maintaining the integrity and functionality of the matrix, as it allows the immobilized agents to effectively hybridize with and capture mtDNA from the blood or plasma. This stable attachment enhances the efficiency and reliability of the mtDNA removal process.

"Crosslinking" refers to the chemical process used to form stable covalent bonds between the binding agents, such as oligonucleotides or polypeptides, and the solid substrate within the matrix of an extracorporeal blood treatment device. This process involves the use of crosslinking agents or chemicals, such as EDC/NHS (1-ethyl-3-(3-dimethylaminopropyl)carbodiimide/N-hydroxysulfosuccinimide), glutaraldehyde, Bis(sulfosuccinimidyl) suberate (BS3), or UVA-irradiation, which facilitate the formation of these covalent bonds. Crosslinking ensures that the binding agents are immobilized on the substrate, allowing them to effectively interact with and mtDNA from the blood or plasma.

According to another embodiment of the invention, the polypeptides and/or oligonucleotides are covalently attached to the support as further detailed below and/or as described in the prior art. Covalent coupling generally includes either covalent non-site-directed attachment of the protein or site-directed attachment of the protein. The support that forms the basis for generating a matrix must provide or facilitate chemical activation, thus allowing the chemical coupling of the enzyme. Many coupling methods for immobilizing enzymes are well-known in the art.

For example, the activation chemistry should be stable over a wide range of pH, buffer conditions, and temperature, resulting in negligible leaching of the enzymes. The coupling method should avoid improper orientation, multisite attachment, or steric hindrance of the polypeptides and/or oligonucleotides. The polypeptide and/or oligonucleotide density per volume of the matrix can be optimized to promote target accessibility and reaction.

The covalent coupling can be carried out via common functional groups, including amines, alcohols, carboxylic acids, aldehydes, and epoxy groups. Carbodiimide compounds can be used to activate carboxylic groups of proteins for direct conjugation to the primary amines on the support surface via amide bonds. The most commonly used carbodiimides are the water-soluble EDC (1-ethyl-3-(-3-dimethylaminopropyl) carbodiimide) for aqueous crosslinking and the water-insoluble DCC (N', N'-dicyclohexyl carbodiimide) for non-aqueous organic synthesis methods.

Alternatively, the supports may carry specific functional groups for coupling a linker and/or enzyme thereto. For example, functionalized resins are commercially available and known to a person with skill in the art. A wide range of coupling chemistries involving primary amines, sulfhydryls, aldehydes, hydroxyls, and carboxylic acids are available in said commercial supports. Examples of commercially available activated resins are CarboLink Coupling resin, Profinity™ Epoxide resin, Affi-Gel 10 and 15, Epoxy-activated Sepharose™ 6B, Tresyl chloride-activated agarose, and Purolite® Lifetech™ methacrylate polymers functionalized with epoxy groups.

According to the invention "extracorporeal blood treatment" refers preferably to the process of removing substances from body fluids through their clearance from flowing blood in a diverted circuit outside the patient's body (extracorporeal). In the context of the present invention, this process involves passing the blood or plasma through a treatment device containing a matrix with immobilized agents that specifically bind to and remove mtDNA from the circulation. This treatment aims to effectively reduce the levels of mtDNA, which are associated with various medical conditions due to their role in triggering inflammatory responses. After the blood is treated to remove mtDNA, it is returned to the patient's circulatory system.

As used herein, "contacting mtDNA with the matrix" refers to the process of bringing mtDNA in a sample, preferably in blood or plasma, into close proximity with the matrix within an extracorporeal blood treatment device, enabling the mtDNA and the matrix to come into physical contact. This matrix contains immobilized binding agents, such as oligonucleotides or polypeptides, that are specifically designed to hybridize with and capture mtDNA. During the extracorporeal treatment, blood or plasma from a patient is circulated through the device, allowing the mtDNA present in the fluid to interact with the matrix physically. This interaction facilitates the binding of mtDNA to the immobilized agents, enabling its effective removal from the bloodstream.

"Mitochondrial transcription factor A (TFAM)" is a nuclear-encoded protein essential for mitochondrial DNA (mtDNA) maintenance and transcription. TFAM binds mtDNA in both sequence-specific and non-specific manners, regulating transcription initiation, replication, and genome packaging. Sequence-specific binding occurs at mitochondrial promoters, facilitating transcription and priming DNA replication. Non-specific binding compacts and stabilizes mtDNA into nucleoid-like structures. TFAM-induced DNA bending enhances mitochondrial transcription initiation by recruiting transcriptional machinery, including POLRMT and TFB2M. Decreased TFAM expression has been implicated in mtDNA stress signaling and the progression of various diseases. Reduced TFAM levels, as observed in heterozygous cells, disrupt mtDNA packaging, allowing fragmented mtDNA to activate the cGAS-STING pathway and induce pro-inflammatory responses. This signaling contributes to resistance against viral infections but may exacerbate inflammatory environments in diseases. mtDNA mutations, deletions, and depletion-linked to oxidative damage-are associated with neurodegenerative disorders such as Parkinson's, Alzheimer's, and Huntington's diseases, as well as aging. Protective effects of TFAM overexpression have been observed in models of metabolic, cardiovascular, and neurological conditions, although mechanisms remain unclear. Genetic polymorphisms in the TFAM gene have also been associated with neurodegenerative diseases. Collectively, alterations in TFAM and mtDNA dynamics underscore their potential roles in disease pathogenesis and progression.

"DNA polymerase subunit gamma (POLG)" is the catalytic subunit of mitochondrial DNA polymerase, a key enzyme responsible for the replication and repair of mitochondrial DNA (mtDNA). Encoded by the POLG gene on chromosome 15q25, this protein functions as part of a heterotrimeric complex with two accessory subunits encoded by the POLG2 gene. POLG exhibits 5' to 3' polymerase activity, 3' to 5' exonuclease proofreading activity, and 5'-deoxyribose phosphate lyase activity, essential for base excision repair. It ensures the fidelity of mtDNA replication and supports mitochondrial genome maintenance. Mutations in POLG are associated with various mitochondrial disorders, including Alpers-Huttenlocher syndrome (AHS), progressive external ophthalmoplegia (PEO), and mitochondrial neuro-gastrointestinal encephalopathy syndrome (MNGIE), among others. These conditions present with a spectrum of overlapping phenotypes, often involving severe neurological and systemic manifestations, and arise from mtDNA depletion or deletion.

"Mitochondrial single-stranded DNA-binding protein (mtSSB)" is a tetrameric protein essential for replicating, maintaining, and repairing mitochondrial DNA. It binds to single-stranded DNA intermediates, stabilizing them during replication and protecting against degradation. mtSSB interacts with key components of the mtDNA replication machinery, including mitochondrial DNA polymerase γ (POLγ), mitochondrial helicase Twinkle, and other factors involved in mtDNA metabolism. It is involved in primer formation at replication origins, modulating the transition from transcription to replication, and ensuring accurate mtDNA replication. Mutations in mtSSB or its regulatory pathways are linked to mitochondrial disorders, including MELAS and Leigh syndrome.

"Twinkle," a mitochondrial helicase encoded by the TWNK gene, plays a critical role in the replication and maintenance of mitochondrial DNA. This enzyme functions as a hexameric or heptameric DNA helicase, unwinding mtDNA in a 5' to 3' direction, essential for proper mtDNA replication. Twinkle's bifunctional nature allows it to both unwind and recombine DNA. It interacts with other key components of the mitochondrial replisome, such as mitochondrial polymerase $\gamma$ and single-stranded DNA-binding proteins. Mutations in the TWNK gene are associated with several mitochondrial diseases, including progressive external ophthalmoplegia (PEO) and infantile-onset spinocerebellar ataxia (IOSCA), due to mtDNA replication and maintenance defects. These mutations often result in mtDNA deletions, leading to mitochondrial dysfunction and associated neuromuscular disorders.

The protein "C17orf80" has recently been identified as a key player in maintaining mitochondrial DNA homeostasis, which is crucial for mitochondrial function and energy metabolism. C17orf80 was identified in the vicinity of the mitochondrial replication machinery through proximity-based proteomics. The protein is primarily located in mitochondrial nucleoid foci, binding to mtDNA without sequence specificity. This binding suggests that C17orf80 plays a role in mtDNA maintenance, likely by influencing replication processes. Studies show that overexpression of C17orf80 leads to an increase in mtDNA copy number, while its depletion results in reduced mtDNA levels, indicating its critical function in regulating mtDNA replication. Furthermore, C17orf80 was found to affect mitochondrial metabolism and cell proliferation. Its loss impairs mitochondrial function, suggesting it is essential for maintaining mitochondrial health. Despite its pivotal role in mtDNA maintenance, C17orf80 does not appear to be required for the expression of mitochondrial genes under normal conditions. Furthermore, genetic studies have linked mutations in C17orf80 to conditions such as autism spectrum disorder and cancers, though its specific molecular functions remain poorly understood.

In preferred embodiments, the polypeptide binding to an mtDNA is mitochondrial transcription factor A (TFAM) and/or mtDNA-binding fragments thereof. In another preferred embodiment, the polypeptide binding to an mtDNA is DNA polymerase subunit gamma (POLG) and/or mtDNA-binding fragments thereof. In another preferred embodiment, the polypeptide binding to an mtDNA is mitochondrial single-stranded DNA-binding protein (mtSSB) and/or mtDNA-binding fragments thereof. In another preferred embodiment, the polypeptide binding to an mtDNA is Twinkle and/or mtDNA-binding fragments thereof. In another preferred embodiment, the polypeptide binding to an mtDNA is C17orf80 and/or mtDNA-binding fragments thereof.

In further embodiments, mitochondrial transcription factor A (TFAM) and/or mtDNA-binding fragments thereof bind and remove cell-free mtDNA. In further embodiments, DNA polymerase subunit gamma (POLG) and/or mtDNA-binding fragments thereof bind and remove cell-free mtDNA. In further embodiments, mitochondrial single-stranded DNA-binding protein (mtSSB) and/or mtDNA-binding fragments thereof bind and remove cell-free mtDNA. In further embodiments, Twinkle and/or mtDNA-binding fragments thereof bind and remove cell-free mtDNA. In further embodiments, C17orf80 and/or mtDNA-binding fragments thereof bind and remove cell-free mtDNA.

In one embodiment, mitochondrial transcription factor A (TFAM), DNA polymerase subunit gamma (POLG), single-stranded DNA-binding protein (mtSSB), Twinkle, and/or C17orf80, and/or mtDNA-binding fragments thereof bind and remove cell-free mtDNA.

### Diseases to be treated

As used herein, a "medical condition associated with cf-mtDNA" refers to any disease or health disorder that is linked to elevated levels of cell-free mitochondrial DNA (cf-mtDNA) in the blood or plasma, for example, elevated over levels in healthy patients. Such elevated levels may be determined by a skilled person using routine techniques. cf-mtDNA is typically released from cells under stress or damage and resembles bacterial DNA, which can trigger inflammatory responses in the body. Such conditions may include but are not limited to, inflammatory diseases, metabolic disorders, cardiovascular diseases, autoimmune diseases, neurodegenerative disorders, infections, cancer, psychological disorders, and drug-induced toxicity, e.g. chemotherapy-induced neuropathy. The presence of cf-mtDNA in circulation is believed to contribute to or exacerbate these conditions by activating immune pathways, such as the TLR-9 pathway, leading to inflammation and disease progression. The invention aims to mitigate these effects by removing cf-mtDNA, potentially alleviating symptoms and improving patient outcomes.

"Inflammation" is a fundamental biological response initiated by the body's tissues in reaction to harmful stimuli like pathogens, injury, or irritants. It involves a complex interplay of immune cells, blood vessels, and molecular mediators to eliminate the initial cause of injury, clear damaged cells and tissues, and initiate tissue repair. Acute inflammation is the immediate response to injury, involving increased movement of leukocytes into the affected tissues. Chronic inflammation, on the other hand, persists over months or years, leading to a shift in cell types present at the site of inflammation and simultaneous tissue destruction and repair. While acute inflammation is a protective mechanism, chronic inflammation is associated with various diseases like atherosclerosis, arthritis, and allergies. Inflammation can be classified based on the type of cytokines and helper T cells involved, and it is essential to distinguish between inflammation and infection, as they are often correlated but can have different underlying causes. Causes of inflammation include physical, biological, chemical, and psychological factors, with acute inflammation typically triggered by exposure to substances, injury, or infection. In contrast, chronic inflammation may result from hypersensitivity, autoimmune disorders, or persistent acute inflammation.

"Autoimmune disorders" or "immunological diseases," refer to conditions in which the immune system malfunctions, either by overactivity (autoimmune disorders) or underactivity (immunodeficiency disorders). Non-limiting examples of autoimmune disorders are Rheumatoid arthritis, Lupus (Systemic Lupus Erythematosus), Lupus nephritis, Multiple sclerosis, Guillain-Barré syndrome, chronic inflammatory demyelinating polyneuropathy (CIPD), Myasthenia gravis, Goodpasture's syndrome, and Pemphigus.

"Hematological disorders" encompass various conditions that affect the blood and blood-forming organs, including red blood cells, white blood cells, platelets, bone marrow, lymph nodes, and spleen. These disorders can be genetic or acquired and may manifest as anemia, clotting disorders, or abnormalities in blood cell production. Examples include but are not limited to sickle cell disease, thalassemia, hemophilia, and von Willebrand disease. Symptoms vary depending on the disorder but may include fatigue, weakness, abnormal bleeding or bruising, and increased susceptibility to infections. Diagnosis typically involves blood tests and sometimes a bone marrow biopsy. Treatment options include medications, blood transfusions, and surgical interventions such as splenectomy.

"Degenerative diseases" encompass a spectrum of conditions marked by the progressive deterioration of tissues or organs over time. These diseases can affect various bodily functions, including movement, cognition, and organ function. While some degenerative diseases have known causes, such as genetic factors or environmental toxins, others may arise spontaneously without a clear etiology. Neurodegenerative diseases, in particular, involve the malfunctioning or death of cells within the central nervous system, leading to debilitating conditions such as Alzheimer's disease and Parkinson's disease. Non-limiting examples of other degenerative diseases include various forms of muscular dystrophy, cardiovascular diseases like atherosclerosis, and certain cancers. Although treatments may help manage symptoms, many degenerative diseases lack a cure, emphasizing the importance of comprehensive care and management plans tailored to individual needs. Thus, in embodiments, the present invention enables the treatment of aforementioned degenerative diseases.

"Cancer" is a term that refers to a group of diseases in which abnormal or transformed cells divide without control and can invade nearby tissues, including disease forms where cancer cells spread to other parts of the body through the blood and lymph systems. cfDNA, which originates from such cancer cells or tumor microenvironment and may circulate in the blood of a patient, is known to have the potential to enter neighboring or distal cells and may be capable of altering the biology of recipient cells. This phenomenon has been implicated in the oncogenic transformation of normal cells and the development of metastases (Bronkhorst et al. (2019), Biomolecular Detection and Quantification 17:100087).

As used herein, "metabolic disease" refers to a group of diseases or medical conditions that affect the normal metabolism, the chemical processes on a cellular level to maintain life. These disorders often arise from genetic mutations, enzyme deficiencies, or environmental factors that impair the body's ability to process or transport key molecules such as proteins, carbohydrates, or lipids, resulting in metabolic imbalances. Non-limiting examples include vitamin deficiencies (e.g., vitamins C and D), hormonal imbalances (e.g., postmenopausal osteoporosis, diabetes mellitus), and disorders linked to abnormal nutrient levels. The consequences of said metabolic imbalances can range from mild symptoms to severe outcomes, such as intellectual disability, seizures, muscle weakness, organ failure, blindness, and deafness. The treatment options depend on the specific condition and typically include dietary adjustments (e.g., reduced sugar for diabetes), supplementation (e.g., vitamins or trace elements), enzyme replacement therapies, and, in some cases, gene therapy.

As used herein, "cardiovascular disease" refers to a group of diseases or medical conditions that affect the heart and blood vessels, impairing their normal function and circulation of blood throughout the body. Cardiovascular diseases encompass a wide range of disorders, including coronary artery disease, stroke, peripheral arterial disease, and heart failure. These conditions are often triggered by a combination of genetic predispositions, lifestyle factors (e.g., poor diet, physical inactivity, smoking), and underlying medical issues (e.g., hypertension, diabetes). Treatments include medications (e.g., statins, antihypertensives), surgical interventions (e.g., bypass surgery, stents), and lifestyle changes (e.g., diet, exercise, smoking cessation). Vascular and/or cardiac disease are, without limitation, preferably selected from the group consisting of coronary arterial disease, peripheral atrial disease, cerebral arterial disease, heart failure, dyslipidemias, arterial hypertension, pulmonary hypertension, eclampsia, digital ulcers, erectile dysfunction, migraines, traumatic vascular injuries, transplant vasculopathy, microvascular injuries, myocardial remodeling and vascular remodeling, ischemia-reperfusion injuries and/or vasospastic diseases, or other vascular or cardiac disease associated with mtDNA.

"Neurodegeneration," also referred to as "neurodegenerative disease," refers to a group of disorders characterized by the progressive loss of structure or function of neurons, ultimately leading to their death. Neurodegeneration is often caused by genetic mutations, environmental factors, or age-related processes that disrupt normal cellular homeostasis in the nervous system. Examples of neurodegenerative diseases include Alzheimer's disease, Parkinson's disease, amyotrophic lateral sclerosis (ALS), and Huntington's disease. Current treatments mainly aim to manage symptoms and slow the progression.

"Infection" refers to a condition caused by the invasion and multiplication of pathogenic microorganisms such as bacteria, viruses, fungi, or parasites in the host organism. Infections may result from direct contact, airborne transmission, contaminated surfaces, or vector organisms, often leading to immune responses that manifest as localized or systemic symptoms. Non-limiting examples of infections include bacterial pneumonia, viral influenza, fungal candidiasis, and parasitic malaria. Treatment approaches depend on the causative agent and include antibiotics, antiviral drugs, antifungal medications, antiparasitic agents, and supportive therapies such as hydration and antipyretics.

As used herein, the term "Psychological disease" refers to a group of conditions that significantly affect an individual's emotional, cognitive, or behavioral functioning, affecting their subjective quality of life or objectively their daily activities. These disorders are often caused by genetic, biological, environmental, and psychological factors. Non-limiting examples include depression, anxiety, bipolar disorder, and schizophrenia. Treatment options commonly include a combination of pharmacological interventions (e.g., antidepressants, anxiolytics, antipsychotics), psychotherapy, and lifestyle interventions.

_Treatment_

The invention includes devices that are configured to be located in an extracorporeal blood circuit through which the blood of a patient passes and which comprises means for transporting blood from the patient's vascular system to a blood treatment device at a defined flow rate and then returning the treated blood to the patient, and wherein the device is further configured to reduce levels of mtDNA in the blood or plasma.

According to the invention, the expression "extracorporeal blood treatment" refers preferably to the process of removing mtDNA from body fluids through their clearance from flowing blood in a diverted circuit outside the patient's body (extracorporeal).

Therapeutic procedures include hemodialysis, including intermittent hemodialysis (HD, HDF, HF) and continuous renal replacement therapy (CRRT); hemoperfusion; plasma exchange; and therapeutic apheresis. Such methods are known to a skilled person, and the device of the invention can be incorporated accordingly.

The expression "blood," as used herein, refers to whole blood, which contains all components of an organism's blood, including red cells, white cells, and platelets suspended in plasma. The expression "blood plasma" refers to the fluid, composed of about 92% water, 7% proteins such as albumin, gamma globulin, fibrinogen, complement factors, clotting factors, and 1% mineral salts, sugars, fats, electrolytes, hormones and vitamins which forms part of whole blood but no longer contains red and white cells and platelets. In the context of the present invention, the expression "blood plasma" or "plasma" refers to specific fractions of the above-defined blood plasma in its standard meaning, such as, for example, blood serum.

In therapeutic apheresis, whole blood can be treated, or blood is separated into its component fractions, for example, by centrifugation or using a plasma membrane or filter, and the fraction containing the solute, which shall be removed, is specifically treated prior to return to the patient.

The present invention provides for an apheresis treatment in which whole blood or plasma (containing the target to be removed) is removed from the patient's flowing blood and returned to the patient after having been contacted with a device or matrix, according to the invention. Typical blood or plasma flow rates in an extracorporeal circuit wherein the blood treatment device is perfused with whole blood or plasma is in the range of between 30 ml/min and 200 ml/min, or 7 ml/min and 50 ml/min, respectively. The circuit can be operated in different treatment modes depending on the medical need, including hemodialysis, hemodiafiltration, and hemofiltration mode.

As used herein, the terms "individual", "patient", and "subject" are often used interchangeably and refer to a human or animal that exhibits a symptom of a disease, disorder, or condition that can be treated with the whole blood compatible matrix, the ligand coupled thereto, and/or the blood treatment device comprising same. In preferred embodiments, a subject includes a human being or animal that exhibits symptoms of a disease, disorder, or condition that can be treated with methods disclosed herein.

As used herein, the terms "(medical) disease," "(medical) disorder," and "(medical) condition" are often used interchangeably.

As used herein, "treatment" or "treating" includes any beneficial or desirable effect on the symptoms or pathology of a disease or pathological condition and may include even minimal reductions in one or more measurable markers of the disease or condition being treated. Treatment can optionally involve either the reduction or amelioration of symptoms of the disease or condition or the delaying of the progression of the disease or condition. "Treatment" does not necessarily indicate complete eradication or cure of the disease, condition, or associated symptoms. The phrase "therapeutically effective" is intended to include, within the scope of sound medical judgment, excessive toxicity, irritation, and/or other problems or complications but commensurate with a reasonable benefit/risk ratio.

As used herein, "prevent" and similar words such as "prevented", "preventing", or "prophylactic", etc., indicate an approach for preventing, inhibiting, or reducing the likelihood of the occurrence or recurrence of a disease or condition. It also refers to delaying the onset or recurrence of a disease or condition or delaying the occurrence or recurrence of the symptoms of a disease or condition. As used herein, "prevention" and similar words also include reducing the intensity, effect, symptoms, and/or burden of a disease or condition prior to the onset or recurrence of the disease or condition. In the context of the present invention, mtDNA can serve as a biomarker for mitochondrial dysfunction. Consequently, the selective removal of mtDNA from blood or plasma presents a potential strategy for both monitoring and managing diseases associated with mitochondrial dysfunction. By reducing circulating mtDNA levels, this approach may facilitate the evaluation of mitochondrial health and contribute to therapeutic interventions targeting conditions linked to mitochondrial impairment.

The instant disclosure also includes kits, sets, packages, and multi-container units containing the herein-described whole blood compatible matrix, the apheresis matrix, and/or the device comprising the same, and further including, where applicable, means for mounting same on equipment for providing extracorporeal blood treatment to a subject in need.

### General Remarks

All words and terms used herein shall have the same meaning commonly given to them by the person skilled in the art unless the context indicates a different meaning. All terms used in the singular shall include the plural of that term and vice versa.

It will be understood that particular embodiments described herein are shown by way of illustration and not as limitations of the invention. The principal features of this invention can be employed in various embodiments without departing from the scope of the invention. Those skilled in the art will recognize or be able to ascertain, using most routine study, numerous equivalents to the specific procedures described herein. Such equivalents are considered to be within the scope of this invention and are covered by the claims. All publications and patent applications mentioned in the specification indicate the skill level of those skilled in the art to which this invention pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more," "at least one," and "one or more than one." The term "or" in the claims is used to mean "and/or" unless explicitly indicated to refer to alternatives only or the alternatives are mutually exclusive. However, the disclosure supports a definition of only alternatives and "and/or." Throughout this application, where relevant, the term "about" indicates that a value includes the inherent variation of error for the device, the method employed to determine the value or the variation among the study subjects.

### FIGURES

The invention is demonstrated by way of example in the following figures. The figures are to provide a further description of potentially preferred embodiments that enhance the support of one or more non-limiting embodiments of the invention.

### Brief description of the figures:

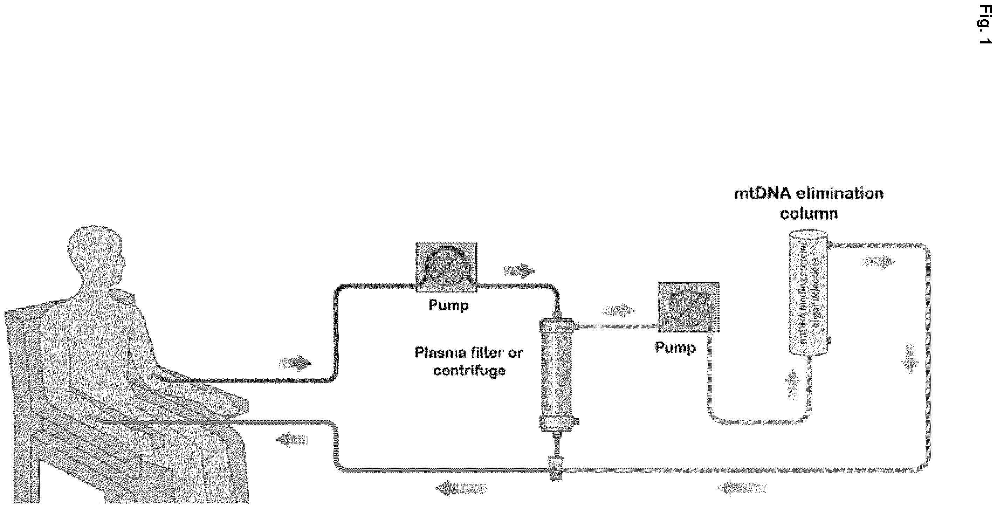

**Figure 1:** Process of Extracorporeal Blood Treatment for mtDNA Removal.

### Detailed description of the figures:

**Figure 1: Process of Extracorporeal Blood Treatment for mtDNA Removal.** Blood is drawn from the patient through venous access, anticoagulated, and separated into its cellular and liquid components using centrifugation or filtration. The resulting plasma is passed through an elimination column containing proteins that bind mitochondrial DNA (mtDNA) or oligonucleotides specific to mtDNA. The mtDNA-depleted plasma is then recombined with the cellular blood components and returned to the patient through a blood return line.

### EXAMPLES

The invention is demonstrated by way of the examples disclosed below. The examples provide technical support for and a more detailed description of potentially preferred, non-limiting embodiments of the invention.

### Summary of the Examples

In order to demonstrate the functionality and beneficial properties of the matrix described herein, the following examples are to be considered:

***Example 1: Methodology***

Recombinant proteins including TFAM, SSBP1, POLRMT, DHX30, DDX28, HADHA, SHMT2, POLG, POLG2, TFB2M, TFB1M, MTERF, PE01, SUPV3L1, HSPA1, HSPD1, PHB2, ATAD3, ALC25A5, CPT1A, DNAJ3, NDUFS1, LRPPRC, ATB5B, SLC25A5, VDAC1, VDAC2, NDUFA9, CLPX, HADHB, TOP1MT, TUFM, NDUFS3, CPS1, PDIP38, DBT, LRRC59, MGC5352, IMMT, KTN1, RPN1, LONP1, and MTERF2 (or other proteins with at least 80% similarity in amino acid sequence) are produced in bacterial systems using vectors suitable for overexpression or other recombinant protein production technologies, followed by purification. The purified proteins are then immobilized on a column using standard coupling methods, such as sulfhydryl coupling resin, amino coupling resin, or NHS-activated resin. Alternatively, specific oligonucleotides are immobilized on the column to utilize the ability of single-stranded DNA (ssDNA) to hybridize via hydrogen bonding with its complementary sequence. For this purpose, human mitochondrial DNA (mtDNA) sequences spanning the 16.5 kb genome are synthesized as 15-20 bp overlapping amplicons from both strands and immobilized on sepharose beads using standard crosslinking methods, such as the EDC/NHS (1-ethyl-3-(3-dimethylamino)propyl carbodiimide/N-hydroxysulfosuccinimide) coupling method.

*In vitro* experiments are performed using purified proteins and plasma or whole blood obtained from healthy volunteers to assess the efficacy of the method. Total DNA is first extracted from the samples using commercially available DNA extraction kits, and the concentration and quality are assessed using a NanoDrop ND1000 spectrophotometer. The DNA concentration is adjusted to 10 ng/$\mu$L and used either in its native form or after sonication to avoid dilution bias. Mitochondrial DNA (mtDNA) copy number and nuclear DNA content are quantified using absolute quantification via real-time quantitative PCR (qPCR). The method involves specific primers targeting the mitochondrial non-coding region and nuclear beta-2-microglobulin (B2M) gene. Specifically, 2 $\mu$L of sample DNA (sonicated or neat) is loaded in triplicate wells with 8 $\mu$L of Master Mix containing 1x QuantiFast SYBR Green Master Mix and 500 nM forward and reverse primers (hMito forward: 5'-CACTTTCCACACAGACATCA-3'; hMito reverse: 5'-TGGTTAGGCTGGTGTTAGGG-3'; hB2M forward: 5'-TGTTCCTGCTGGGTAGCTCT-3'; hB2M reverse: 5'-CCTCCATGATGCTGCTTACA-3'). A five-point standard curve, generated by 10-fold serial dilutions of known copy numbers of mitochondrial and nuclear DNA, is loaded in duplicate on each plate. For cellular samples such as whole blood or PBMCs, the mtDNA copy number (mtDNA-CN) is normalized to B2M to determine the absolute mtDNA content per cell (Mt/N), calculated as the mtDNA copy number divided by the B2M copy number and multiplied by 2 to account for the diploid nuclear genome. In cell-free samples like plasma or serum, mtDNA and B2M copy numbers are adjusted for the starting sample volume and final elution volume using the equation:

$$\text{Copy number} / \mu L = \frac{(\text{Copy number per 2}\mu\text{L reaction} \times \text{ Elution volume } (\mu L))}{\text{qPCR reaction volume } (\mu L) \times \text{ Sample volume } (\mu L)}$$

Additional methods for assessing mtDNA-CN include relative qPCR using the $\Delta\Delta$CT method, TaqMan probe-based technologies, digital PCR, as well as estimation from whole genome sequencing and microarray data. Development of high-throughput approaches is ongoing.

For clinical applications, venous blood is drawn from the patient, anticoagulated, and separated into cellular and liquid components using centrifugation or filtration. The separated plasma is perfused through an elimination column containing mtDNA-binding proteins or specific oligonucleotides immobilized on a matrix. The mtDNA-depleted plasma is then recombined with the cellular blood components and returned to the patient through a blood return line.

**REFERENCES**

1. Malik, A.N., et al., Mitochondrial DNA as a non-invasive biomarker: accurate quantification using real time quantitative PCR without co-amplification of pseudogenes and dilution bias. Biochem Biophys Res Commun, 2011. 412(1): p. 1-7.

2. Rosa, H.S., et al., A case for measuring both cellular and cell-free mitochondrial DNA as a disease biomarker in human blood. FASEB J, 2020. 34(9): p. 12278-12288.

**Claims**

1. A matrix configured for use in an extracorporeal blood treatment device, wherein the matrix comprises a solid substrate to which an agent is immobilized, wherein said agent specifically binds mitochondrial DNA (mtDNA) in the blood or blood plasma of a subject.

2. The matrix according to claim 1, wherein the matrix is configured to bind and remove cell-free (cf-mtDNA) from human blood or blood plasma by extracorporeal treatment.

3. The matrix according to claim 1 or 2, wherein the agent comprises an oligonucleotide and/or a polypeptide that specifically binds mtDNA.

4. The matrix according to claim 3, wherein the agent comprises multiple oligonucleotides of different nucleic acid sequences, each hybridizing to a mtDNA sequence.

5. The matrix according to claim 3 or 4, wherein the oligonucleotide is a single-stranded DNA (ssDNA) oligonucleotide.

6. The matrix according to any one of claims 3 to 5, wherein the oligonucleotide has a length of 10 to 50 nt, preferably 12 to 30 nt.

7. The matrix according to any one of claims 3 to 6, wherein the oligonucleotide is obtained by synthesizing multiple mtDNA sequences of a human mtDNA genome, wherein the mtDNA sequences are synthesized as overlapping amplicons from the sense and antisense strand of said human mtDNA genome.

8. The matrix according to claim 3, wherein the polypeptide specifically binds to human mtDNA and is a protein selected from the group consisting of TFAM, POLG, mtSSB (mitochondrial single stranded DNA binding protein), Twinkle (mtDNA helicase), C17orf80, SSBP1, POLRMT, DHX30, DDX28,HADHA, SHMT2, POLG, POLG2, TFB2M, TFB1M, MTERF, PE01, SUPV3L1, HSPA1, HSPD1, PHB2, ATAD3, ALC25A5, CPT1A, DNAJ3, NDUFS1, LRPPRC, ATB5B, SLC25A5, VDAC1, VDAC2, NDUFA9, CLPX, HADHB, TOP1MT, TUFM, NDUFS3, CPS1, PDIP38, DBT, LRRC59, MGC5352, IMMT, KTN1, RPN1, C17orf80, Mgm101p, MTERF1, LONP1 and MTERF2, preferably TFAM, POLG, mtSSB (mitochondrial single stranded DNA binding protein), Twinkle (mtDNA helicase), C17orf80, and/or mtDNA-binding fragments thereof.

9. A method for preparing the matrix according to any one of the preceding claims, wherein the oligonucleotide according to claims 3 to 7 and/or the polypeptide according to claims 3 or 10 is immobilized on the solid substrate of the matrix by covalent coupling, preferably by crosslinking, for example by EDC/NHS (1-ethyl-3-(3-dimethylamino) propyl carbodiimide/N-hydroxysulfosuccinimide) treatment.

10. A blood treatment device configured to remove mtDNA from the blood or blood plasma of a person in need thereof in an extracorporeal blood circuit, wherein the device comprises a matrix according to any one of claims 1 to 8.

11. An extracorporeal blood circuit, comprising a blood treatment device according to claim 10, wherein the extracorporeal blood circuit comprises means for transporting blood or blood plasma from a patient's vascular system to the blood treatment device at a defined flow rate and means for returning the treated blood or blood plasma back to the patient.

12. The extracorporeal blood circuit according to claim 11, wherein the extracorporeal blood circuit in which the blood treatment device is located further comprises a plasma separator, such as a plasma filter or centrifuge-based plasma separation system or combination thereof, which allows for the separation of plasma from the blood, and wherein the blood treatment device is located downstream of a plasma outlet of the plasma separator.

13. An *ex vivo* method for binding mtDNA comprising contacting mtDNA with the matrix according to any one of claims 1 to 8.

14. An agent that specifically binds mitochondrial DNA (mtDNA), as described in any one of claims 3 to 8, for use in the treatment and/or prevention of a medical condition associated with cf-mtDNA, wherein the agent is immobilized to a solid substrate of a matrix, wherein said matrix is configured for specifically binding and removing cf-mtDNA from the blood or blood plasma of a subject, wherein said matrix is present in a blood treatment device according to claim 10, and wherein said device is present in an extracorporeal blood circuit transporting blood or blood plasma from a patient's vascular system to the blood treatment device and returning the treated blood or blood plasma back to the patient.

15. The agent for use according to claim 14, wherein the medical condition is selected from the group consisting of an inflammatory disease, a metabolic disorder, a cardiovascular disease, an autoimmune disease, neurodegeneration, an infection, cancer, and a psychological disorder.

**Fig. 1**

Europäisches
Patentamt

European
Patent Office

Office européen
des brevets

## EUROPEAN SEARCH REPORT

**Application Number**

EP 25 15 5160

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/108637 A1 (CEDARS SINAI MEDICAL CENTER [US]; BORAH SUPRIYA [US]) 3 June 2021 (2021-06-03) * paragraphs [0130], [0133], [0137], [0141], [0145], [0197] * | 1-15 | INV. G01N33/579 A61M1/00 A61P35/00 A61P37/00 |
| A | WO 2022/058604 A1 (BELGIAN VOLITION SRL [BE]) 24 March 2022 (2022-03-24) * claim 12; figure 1 * | 1-15 | |
| A | WO 2023/126672 A1 (SANTERSUS AG [CH]) 6 July 2023 (2023-07-06) * paragraphs [0009], [0010], [0019] * | 1-15 | |
| A | TUMBURU LAXMINATH ET AL: "Cell-Free Mitochondrial DNA Is Elevated in Sickle Cell Disease Patients, and Serve As a Potential Proinflammatory DAMP", BLOOD, ELSEVIER, AMSTERDAM, NL, vol. 132, 29 November 2018 (2018-11-29), page 1068, XP086594244, ISSN: 0006-4971, DOI: 10.1182/BLOOD-2018-99-118440 * the whole document * | 1-15 | TECHNICAL FIELDS SEARCHED (IPC) A61P A61M G01N |
| A | ZHONG FEI ET AL: "Emerging Role of Mitochondrial DNA as a Major Driver of Inflammation and Disease Progression", TRENDS IN IMMUNOLOGY, ELSEVIER LTD. TRENDS JOURNALS, GB, vol. 40, no. 12, 16 November 2019 (2019-11-16), pages 1120-1133, XP085925697, ISSN: 1471-4906, DOI: 10.1016/J.IT.2019.10.008 [retrieved on 2019-11-16] * the whole document * | 1-15 | |

The present search report has been drawn up for all claims

| Place of search | Date of completion of the search | Examiner |
|---|---|---|
| Munich | 2 July 2025 | Cilensek, Zoran |

EPO FORM 1503 03.82 (P04C01)

## ANNEX TO THE EUROPEAN SEARCH REPORT
## ON EUROPEAN PATENT APPLICATION NO.

EP 25 15 5160

This annex lists the patent family members relating to the patent documents cited in the above-mentioned European search report.
The members are as contained in the European Patent Office EDP file on
The European Patent Office is in no way liable for these particulars which are merely given for the purpose of information.

02-07-2025

| Patent document cited in search report | Publication date | Patent family member(s) | | Publication date |
|---|---|---|---|---|
| WO 2021108637 A1 | 03-06-2021 | CA | 3162518 A1 | 03-06-2021 |
| | | EP | 4065599 A1 | 05-10-2022 |
| | | JP | 2023503615 A | 31-01-2023 |
| | | US | 2023121867 A1 | 20-04-2023 |
| | | WO | 2021108637 A1 | 03-06-2021 |
| WO 2022058604 A1 | 24-03-2022 | EP | 4213909 A1 | 26-07-2023 |
| | | JP | 2023544112 A | 20-10-2023 |
| | | US | 2023355851 A1 | 09-11-2023 |
| | | WO | 2022058604 A1 | 24-03-2022 |
| WO 2023126672 A1 | 06-07-2023 | US | 2023201439 A1 | 29-06-2023 |
| | | WO | 2023126672 A1 | 06-07-2023 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021108637 A1

**Non-patent literature cited in the description**

- **MALIK, A.N. et al.** Mitochondrial DNA as a non-invasive biomarker: accurate quantification using real time quantitative PCR without co-amplification of pseudogenes and dilution bias. *Biochem Biophys Res Commun*, 2011, vol. 412 (1), 1-7

- **ROSA, H.S. et al.** A case for measuring both cellular and cell-free mitochondrial DNA as a disease biomarker in human blood. *FASEB J*, 2020, vol. 34 (9), 12278-12288